Europäisches Patentamt

European Patent Office

Office européen des brevets

(19)

(11) Publication number: **0 184 369 B1**

(12) **EUROPEAN PATENT SPECIFICATION**

(45) Date of publication of patent specification: **01.04.92** (51) Int. Cl.⁵: **C12P 21/00**, C12N 15/00

(21) Application number: **85308580.1**

(22) Date of filing: **26.11.85**

(54) Monoclonal antibody specific for a mammary tumor cell surface antigen.

(30) Priority: **05.12.84 US 678261**

(43) Date of publication of application:
**11.06.86 Bulletin 86/24**

(45) Publication of the grant of the patent:
**01.04.92 Bulletin 92/14**

(84) Designated Contracting States:
**AT BE CH DE FR GB IT LI LU NL SE**

(56) References cited:
**EP-A- 0 118 365**
**EP-A- 0 160 446**
**US-A- 4 522 918**

**PROC. NATL. ACAD. SCI. USA, vol. 80, March 1983, pages 1332-1336; H.R. SOULE et al.: "Membrane 126-kilodalton phosphoglycoprotein associated with human carcinomas identified by a hybridoma antibody to mammary carcinoma cells"**

(73) Proprietor: **THE SALK INSTITUTE FOR BIO-LOGICAL STUDIES**
**10010 North Torrey Pines Road**
**La Jolla California 92037(US)**

(72) Inventor: **White, Christine Anna**
**1630 Crest Drive**
**Encinitas California 92024(US)**
Inventor: **Dulbecco, Renato**
**7525 Hillside Drive**
**La Jolla California 92037(US)**
Inventor: **Allen, William Ross**
**167 Countryhaven Road**
**Encinitas California 92024(US)**

(74) Representative: **Allard, Susan Joyce et al**
**BOULT, WADE & TENNANT, 27 Furnival Street**
**London EC4A 1PO(GB)**

EP 0 184 369 B1

BIOLOGICAL ABSTRACTS, vol. 79, no. 12, 1985, no. 106303, Biological Abstracts, Inc., Philadelphia, PA, US; J. KOVARIK et al.: "Biochemical and histochemical characteristics of target antigen detected by monoclonal antibody HBCa-12 against a membrane component of human mammary carcinoma cell line", & NEOPLASMA (BRATISL) 31(6) 625-630. 1984

BIOLOGICAL ABSTRACTS, vol. 79, no. 7, 1985, no. 60135, Biological Abstracts, Inc., Philadelphia, PA, US; D. KUFE et al.: "Differential reactivity of a novel monoclonal antibody (DF3) with human malignant vs. benign breast tumors", & HYBRIDOMA 3(3): 223-232. 1984

BIOLOGICAL ABSTRACTS, vol. 79, no. 12, 1985, no. 106323, Biological Abstracts, Inc., Philadelphia, PA, US; A.C. WHITE et al.: "2 monoclonal antibodies selective for human mammary carcinoma", & CANCER RES 45(3): 1337-1343. 1985

BIOLOGICAL ABSTRACTS, vol. 74, no. 12, 1982, no. 84638, Biological Abstracts, Inc., Philadelphia, PA, US; M. NUTI et al.: "A monoclonal antibody (B72.3) defines patterns of distribution of a novel tumor-associated antigen in human mammary carcinoma cell populations", & INT. J. CANCER 29(5): 539-546. 1982

## Description

The present invention is related to monoclonal antibodies and more particularly to monoclonal antibodies reactive with an antigen found on mammary tumor cell surfaces.

Antibodies have long been used in medical diagnosis, e.g., determining blood types, and in biological experimentation. With development of techniques of producing monoclonal antibodies which make it possible to obtain homogenous, highly specific antibodies, Kohler G. and Milstein, C.: (1975) Nature - (London) 256 495-497, the utility of antibodies has been greatly increased. Unlike antibody fractions which were previously available and which were actually heterogeneous mixtures of a number of antibody molecules reactive with a variety of antigenic determinants, the molecules in a monoclonal antibody are all identical and generally are reactive with a single antigenic determinant or a group of closely related antigenic determinants. Monoclonal antibodies are therefore much more precise probes for detecting the presence of a particular substance than were previous heterogeneous antibody fractions. The precise selectivity of monoclonal antibodies makes them particularly useful for diagnostic purposes and even as therapeutic agents against selected biological material, such as tumor cells. Monoclonal antibodies have been used to detect and isolate biological substances which were were previously unknown.

Generally, monoclonal antibodies are produced by immunizing an animal with a biological specimen or other foreign substance, obtaining antibody-producing cells from the animal, and fusing the antibody-producing cells with strains of neoplastic cells, e.g., tumor cells, to produce hybridomas which are isolated and cultured as monoclones. The monoclonal hybridomas may either be cultured in vitro or may be grown in vivo as tumors in a host animal. Because each antibody-producing cell produces a single unique antibody, the monoclonal cultures of hybridomas each produce a homogenous antibody fraction which may be obtained either from the culture medium of hybridoma cultures grown in vitro or from the ascitic fluid, or serum of a tumor-bearing host animal.

Not all of the hybridoma clones which result from fusing neoplastic cells with antibody-producing cells are specific for the desired foreign substance or antigen because many of the hybridomas will make antibodies which the animal's immune system has generated in reaction to other foreign substances. Even monoclonal antibodies against the subject antigen will differ from clone to clone because antibodies produced by different clones may react with different antigenic determinants of the same molecule. From each clone, therefore, it is necessary to obtain the resulting antibody or the antibody-containing medium, serum or ascitic fluid and test its reactivity with the subject biological material and to test its specificity by determining what other biological material, if any, it recognizes. While the necessity of characterizing the antibody of each clone adds to the complexity of producing monoclonal antibodies, the wide variety of homogeneous antibodies which may be obtained gives investigators a number of very precise tools to map the structure and development of somatic cells.

A monoclonal antibody is produced which is specific for antigens found predominantly on human mammary tumor cells. Mice are inoculated with human mammary tumor cells, and spleen cells or lymph node cells are obtained from the inoculated mice and fused with mouse tumor cells. Monocultures of the fused cells are produced, and the antibodies obtained from the monoclones are tested for their ability to react with a variety of randomly obtained human mammary tumor tissues. In order to select a monoculture which produces an antibody with the desired characteristics, the reactivity of the antibody with other cells, including both normal human cells and other human tumor cells is investigated. A monoclonal antibody from one hybridoma clone is reactive with a mammary tumor cell surface antigen and is particularly useful for diagnosing mammary tumors. The antibody is potentially useful as an agent against mammary tumors.

Human mammary carcinoma currently represents the leading cause of cancer death in women in the United States with more than 150,000 new cases diagnosed each year. The histopathological classification of human mammary carcinomas is currently dependent on morphologic description alone. Approximately 80% are infiltrating ductal carcinomas, 10% are infiltrating lobular carcinomas, and the remaining 10% comprise a number of histologic types including intraductal carcinoma. Excepting inflammatory carcinoma, for the most part, morphology has not proven to be a good predictor of clinical prognosis or response to therapy. Indeed, extent of disease (tumor size and nodal positivity) have continued to determine clinical management. Recently, with the advent of estrogen and progesterone receptor determinations, correlations have begun to be made between clinical variables and biologic characteristics of malignant mammary cells.

The invention provides for a cell line produced by the fusion of an antibody-producing animal cell and a neoplastic cell, characterised in that it is the hybridoma HB-8655 or an antibody producing reclone thereof. The cell line may be produced wherein the antibody-producing cell is derived from a rodent, for example rodent spleen cells, in particular BALB/c mouse cells, or rodent lymph node cells. The neoplastic cell in the hybridoma production may be a non-antibody producing cell.

The invention also provides for a monoclonal antibody characterised in that it is obtained from HB-8655 hybridoma cultures or antibody-producing reclones thereof and a method of producing such monoclonal antibodies which method comprises inoculating an animal with material derived from the human mammary carcinoma cell line MCF-7

In accordance with the invention, a hybridoma is developed which produces a monoclonal antibody which is generally reactive with human mammary carcinoma cells, specifically reacting with a cell surface antigen that is prevalent on mammary cell tumor surfaces. The antibody reacted with 26 out of 29 randomly obtained human mammary carcinomas tested, reacts more weakly with normal human epithelial cells of breast, renal proximal tubule, skin, esophagus and salivary gland, but cells of substantially no other normal tissue, and was unreactive with 13 of 17 other malignant tissues tested. Because the mammary carcinoma tissues were randomly obtained, the antibody is expected to react with about the same proportion of other randomly obtained human mammary carcinoma tissues. As the antibody detects an antigen found predominantly in human mammary carcinomas, it is useful in determining the cellular lineage from which human mammary carcinomas arise and has potential clinical utility in breast cancer treatment.

A monoclonal antibody to the breast carcinoma cell line MCF-7 was produced utilizing the technique of Kohler and Milstein supra. The mammary tumor cell line MCF-7 (Soule, H.D., et al. JNCI, 51:1409-1413 (1973)), was cultured in DMEM with 10% fetal calf serum and NEAA (8.9 mg/L L-alanine, 15.0 mg/L L-asparagine, 13.3 mg/L L-aspartic acid, 14.7 mg/L L-glutamic acid, 7.5 mg/L L-glycine, 11.5 mg/L L-proline, 10.5 mg/L L-serine).

BALB/c mice were immunized with $10^6$ MCF-7 cells injected intraperitoneally every 3 weeks for a total of 3 to 4 injections. The mice were sacrificed three days after the last injection and their spleens were taken. A spleen cell suspension was prepared, and the resulting cell suspension was washed by two centrifugations (800 x g) in protein-free Dulbecco's modified Eagles medium.

Because the antibody-producing cells obtained from the spleen do not independently reproduce, and thus cannot be cultured, they are fused with cells which may be independently cultured either in vivo or in vitro so that the genetic and metabolic processes of the fused hybridomas have characteristics of each of the parent cells, and it is intended that certain of the cells obtained will have the capability to independently reproduce and to produce the antibody of the antibody-producing parent cell. Some tumor cells, particularly myeloma cells, may be advantageously fused with antibody-producing cells to produce hybridomas. Although it is not necessary, it is preferred that the tumor cells and antibody-producing cells be derived from the same species to enhance the likelihood that the genetic and biochemical properties of the parent cells will be compatible and thus produce viable and stable hybridomas. A number of myeloma cultures have been characterized, and herein, mouse-derived, nonantibody-producing myeloma cell line, PAI that was obtained from Dr. Theo Stachlin, Basil, Switzerland, J. Stocker, Research Disclosure 21713, 155-157 (1982), were used to produce the hybridomas. It is to be understood that other tumor lines, which include but are not limited to P3NS1, Y3, SP2/0 and their derivatives, may also be used.

It is advantageous to select a myeloma line which does not produce antibody so that the resulting hybridoma will only produce the antibody of the parent spleen or lymph node cell. This is particularly important when the antibody is used for therapeutic purposes, e.g., as a cytotoxic agent against tumor cells, where it is undesirable to introduce extraneous antibodies which could produce side reactions.

The myeloma cells are maintained in Dulbecco's modified Eagle's medium supplemented with 10% horse serum. $10^7$ myeloma cells and $10^8$ cells obtained from the immunized mice are resuspended for fusion in a 45% solution (v/v) of polyethelyene glycol 1500. Cell hybrids are selected in hypoxanthine aminopterin thymidine (HAT) medium, all growth in HAT medium being indicative of successful hybridization of mouse spleen and mouse myeloma cells.

Clones of hybridomas may be grown in vitro according to known tissue culture techniques such as is described by Cotten et al., Eur. J. Immunol. 3, 136 (1973). Alternatively, hybridomas may be grown in vivo as tumors in a histocompatible animal or in athymic nude mice. The antibodies may be recovered from the in vitro culture medium (the supernatant of the clone) or from the serum or ascitic fluid of the animal by means known in the art, e.g., Gerhard et al., Proc. Natl. Acad. Sci., 75, pp. 1510-1514 (1978). In some cases it may be advantageous to obtain the antibodies directly from the cells of the culture or tumor.

The initial specificity screening of hybridoma supernatants using dried MCF-7, MDA-157 and DU4475 (other mammary carcinoma cell lines which were cultured in the manner that MCF-7 was cultured) and a human foreskin fibroblast (HFF) cell line as target cells was performed by ELISA assay. Hybrids which reacted with either MCF-7 and/or MDA-157 (but not HFF cells) were chosen for a second screening.

Selected hybridomas were cultured in DMEM supplemented with 10% horse serum, NEAA, $10^{-5}$ M mercaptoethanol.

When a useful hybridoma clone is produced it is generally advantageous to reclone the cell line to avoid overgrowth of cultures with variant cells no longer producing antibody. Because the hybridoma contains some, but not all, of the genetic material of each parent cell, the full characteristics of the hybridoma are not known. Often a hybridoma clone, due to original genetic deficiency or subsequent chromosome loss, after several passages may lose its ability to reproduce and/or to produce the particular antibody. Accordingly, it is important, soon after the initial hybridization, that a hybridoma clone of interest is recloned to insure the availability of functioning strains of the antibody-producing hybridoma. By recloning is meant fusing a hybridoma cell with neoplastic cells, e.g., to isolate individual hybridoma cells and expand them into cultures which are clones.

A cell line culture initially designated as 15A8 and its reclones produce a monoclonal antibody specific for a cell surface antigen that occurs predominantly on human mammary carcinoma cells. The 15A8 cell line is on deposit at the American Tissue Culture Collection of 12301 Parklawn Drive, Rockville, Maryland 20852 and has been assigned the accession number HB-8655.

The isotype of monoclonal antibody 15A8 was determined by Ouchterlony gel immunodiffusion with rabbit antiserum to mouse IgG1, IgG2a, IgG2b, IgG3, IgM and IgA (Miles Laboratory). 15A8 monoclonal antibody was determined to be of the IgG1 isotype. The 15A8 antibody is characterized in that it reacts with a surface antigen present in nearly all breast cancers examined, as well as with all fibrocystic diseases and normal mammary epithelium. It reacts with some other normal tissues and frequently with a number of adenocarcinomas not of breast origin. It does not react with mesotheliomas and is therefore suitable for the differential diagnosis of these cancers from metastatic breast cancer. It slows down the growth of cells of the MDA human breast cancer line in vitro.

The monoclonal antibody was purified to greater than 95% homogeneity by the following steps: ascites was obtained following injection of hybridoma cells into previously pristane-primed BALB/c mice. The resulting ascitic fluid was clarified by centrifugations at 10,000 x g for 10 minutes. 45% ammonium sulfate precipitation was followed by centrifugation at 10,000 x g for 10 minutes. The pellet was resuspended in 10 ml. of 20mM Tris .02% $NaN_3$ pH 8 buffer, dialyzed against the same buffer and applied to an affi-gel blue DEAE ion-exchange column. The column was washed with 3 bed volumes of 20mM Tris plus 28 mM NaCl with .02% $NaN_3$ buffer, and the antibody was eluted with a NaCl gradient. Fractions (2 ml.) were collected and an OD 280 was determined. Immunoperoxidase on human mammary tumors was used to localize fractions containing antibody. The appropriate fractions were pooled. The protein concentration was determined by the method of Lowry et al., J. Biol. Chem. 193, 265-275 (1975). The degree of purification was determined by polyacrylamide gel electrophoresis.

Purified 15A8 antibody (10 mg/ml) was found to be highly reactive at dilutions of 1:10,000 with MCF-7 and MDA-175 cells. By immunofluorescence, the pattern appeared to be that of surface membrane reactivity. The reactivity persisted, although reduced, following methanol-acetic acid fixation.

Portions of fresh normal and malignant tissues were obtained from the Surgical and Anatomical Pathology Departments of the UCSD Hospital and the Veterans Hospital, San Diego. Fresh frozen tissues were obtained from the biological carcinogenesis branch of the National Cancer Institute. The reactivity of the 15A8 monoclonal antibody with the several tissues was determined by immunoperoxidase staining.

Tissues were coated with Tissue Tek OCT Compound (Scientific Products) and frozen at -70°C. Sections of frozen tissue blocks 4 $\mu$M thick were cut on the microtome/cryostat, mounted on glass slides and stored at -70°C. Mounted slides were stained by an indirect immunoperoxidase assay. Briefly, slides were hydrated with PBS, then partially air-dried. The monoclonal antibody was overlayed onto sections and incubated at room temperature for 30 minutes in a humid chamber. Sections were then overlaid with a 1:100 dilution of horseradish peroxidase conjugated with goat anti-mouse immunoglobulin and incubated for 30 minutes. The color reaction was developed with diaminobenzidine (0.6 mg/ml) and 0.03% hydrogen peroxide. Cells were counterstained in hematolxylin, washed in water, dehydrated in 100% ethyl alcohol, cleared in xylene, mounted in Permount, covered with a coverslip and examined using a Zeiss microscope. The reactivities of 15A8 with human mammary carcinoma cell lines and other tumor tissues are shown in Table 1.

## TABLE 1

### BINDING OF 15A8 ANTIBODY TO HUMAN MAMMARY CARCINOMA CELL LINES AND OTHER HUMAN TUMOR TISSUES

|  | 15A8 |
|---|---|
| **CELL LINES (live)** | |
| **Mammary carcinoma** | |
| MCF-7 | + |
| MDA-157 | + |
| DU4475 | − |
| **Non-mammary** | |
| HFF | − |
| PA1 | − |
| **TISSUES (frozen)** | |
| Human mammary carcinoma (total) | + (26/29) |
| Primary infiltrating ductal carcinoma | + (14/17) |
| Infiltrating ductal cancer metastatic to liver, lung omentum and brain | + (5/5) |
| Intraductal papillary, colloid mammary carcinomas | + (6/6) |
| Comedo carcinoma | + (1/1) |
| Cystosarcoma phylloides | − (1) |
| Papillary ductular hyperplasia, sclerosins adenosis | + (2/2) |
| Fibrocystic disease | + (2/2) |
| Fibroadenoma | + (2/2) |
| Normal mammary epithelium | + (4/4) |

-------------------------------------------------------------------

+ = positive immunoperoxidase reaction (usually strong)
− = no reaction
* = weak reaction

Table 2 below summarizes the reactivities of 15A8 with normal tissues and nonmammary malignancies. 15A8 had crossreactivities with normal breast, renal proximal tubule, epidermal, esophageal, and salivary gland epithelium, and with one specimen each of cervical, colon and prostate carcinomas. 15A8 stained 3 of 6 specimens of known estrogen receptor and progresterone receptor negative breast carcinomas, but stained all four specimens of known estrogen receptor and/or progesterone receptor positive breast carcinomas.

## TABLE 2

### BINDING OF ANTIBODY 15A8 TO NORMAL
### TISSUES AND NONMAMMARY MALIGNANCIES

--------------------------------------------------------------

|  | 15A8 |
|---|---|
| **NORMAL TISSUES** | |
| Epidermis | + |
| Salivary gland | + |
| Thyroid | - |
| Adrenal | - |
| Lung | - |
| Bronchus | - |
| Heart | - |
| Aorta | - |
| Esophagus | + |
| Stomach | - |
| Small bowel | - |
| Large bowel | - |
| Liver (2) | - |
| Pancreas | - |
| Gall bladder | - |
| Spleen | - |
| Lymph nodes | - |
| Kidney (2) | prox tubule + |
| Bladder | - |
| Ovary | - |
| Testis | - |
| Cervix | - |
| Uterus | - |
| Bone marrow | - |
| Brain | - |
| **NONMAMMARY MALIGNANCIES** | |
| Lung | |
|   Squamous cell cancer | + |
|   Adenocarcinoma | - |
|   Small cell cancer | - |
| Gastrointestinal | |
|   Gastric cancer | - |
|   Cholangiocarcinoma | - |
|   Pancreatic cancer | - |
|   Colon cancer (2) | -/+ |
| Genito-urinary | |
|   Cervix cancer | + |
|   Ovarian cancer | - |
|   Bladder cancer | - |
|   Renal cancer | - |
|   Prostate cancer (2) | -/+ |
| Lymphoma | |
|   T cell | - |
| Mesothelioma | - |
| Melanoma | - |

Because 15A8 antibody detects antigens found predominatly in human mammary carcinomas, this antibody is presently useful for diagnosing mammary carcinoma cells and should prove useful in future studies of the lineage patterns of these tumor cells. 15A8 may also have application in both tumor localization and therapy because it is exposed at the cell surface. The therapeutic implications are also supported by preliminary evidence that this monoclonal antibody inhibits growth in vitro and in vivo of

7

breast cancer cells.

Of particular significance is the fact that the monoclonal antibody does not react with mesothelioma cells. Although these cells are not mammary tumor cells, they often occur at the same site as metastatic mammary carcinomas, and the 15A8 antibody is therefore particularly useful for distinguishing metastasized carcinomas from mesothelioma tumors.

Modifications obvious to one with ordinary skill in the art may be made without departing from the scope of the present invention. For example, antibody production may be induced in the host animal by inoculating the animal with cell membrane fragments or cell membrane derived material rather than with complete mammary tumor cells.

## Claims
## Claims for the following Contracting States : BE, CH, DE, FR, GB, IT, LI, LU, NL, SE

1. A cell line produced by the fusion of an antibody-producing animal cell and a neoplastic cell, characterised in that it is the hybridoma HB-8655 or an antibody producing reclone thereof.

2. A cell line as claimed in claim 1 wherein the antibody-producing cell is derived from a rodent.

3. A cell line as claimed in claim 2 wherein the antibody-producing cells are rodent spleen cells or rodent lymph node cells.

4. A cell line as claimed in claim 3 wherein the antibody-producing cells are BALB/c mouse spleen cells.

5. A cell line as claimed in any one of the preceding claims wherein the neoplastic cell is a non-antibody producing cell.

6. A monoclonal antibody characterised in that it is obtained from HB-8655 hybridoma cultures or antibody-producing reclones thereof.

7. A method of producing monoclonal antibodies as claimed in claim 6 which method comprises inoculating an animal with material derived from the human mammary carcinoma cell line MCF-7

## Claims for the following Contracting State : AT

1. A method of producing a monoclonal antibody characterised in that it is obtained from HB-8655 hybridoma cultures or antibody reclones thereof which method comprises inoculating an animal with material containing the antigen, obtaining antibody-producing cells from the animal, fusing the antibody-producing cells and neoplastic cells to produce hybridomas, selecting the hybridoma, producing clones of the selected hybrodomas, and collecting the antibody produced thereby.

2. A method as claimed in claim 1 wherein the antibody-producing cells are BALB/c mouse spleen cells.

3. A method as claimed in any one of the preceding claims wherein the antigen-containing material is dervied from the human mammary carcinoma cell line MCF-7.

4. A method as claimed in any one of the preceding claims wherein the neoplastic tissue cells are of the myeloma cell line PAI.

5. A method as claimed in any one of the preceding claims wherein the clones are grown in host animals, and the antibody is obtained from the host animal's blood serum or the host animal's ascitic fluid.

6. A method as claimed in any one of claims 1 to 4 wherein the clones are grown in vitro, and the antibody is obtained from culture supernatants of the clones.

## Revendications
## Revendications pour les Etats contractants suivants : BE, CH, DE, FR, GB, IT, LI, LU, NL, SE

1. Une lignée cellulaire obtenue par la fusion d'une cellule animale productrice d'anticorps et d'une cellule néoplasique, caractérisée en ce que c'est l'hybridome HB-8655 ou un de ses reclones producteur d'anticorps.

2.  Une lignée cellulaire selon la revendication 1 dans laquelle la cellule productrice d'anticorps dérive d'un rongeur.

3.  Une lignée cellulaire selon la revendication 2 dans laquelle les cellules productrices d'anticorps sont des cellules de rate de rongeur ou des cellules de ganglions lymphatiques de rongeur.

4.  Une lignée cellulaire selon la revendication 3 dans laquelle les cellules productrices d'anticorps sont des cellules de rate de souris BALB/C.

5.  Une lignée cellulaire selon l'une quelconque des revendications précédentes dans laquelle la cellule néoplasique est une cellule non productrice d'anticorps.

6.  Un anticorps monoclonal caractérisé en ce qu'il est obtenu à partir de cultures d'hybridome HB-8655 ou de ses reclones producteurs d'anticorps.

7.  Un procédé de production d'anticorps monoclonaux selon la revendication 6, procédé qui comprend l'inoculation à un animal d'un matériel dérivé de la lignée cellulaire de carcinome mammaire humain MCF-7.

**Revendications pour l'Etat contractant suivant : AT**

1.  Un Procédé de production d'un anticorps monoclonal, caractérisé en ce qu'il est obtenu à partir de cultures d'hybridome HB-8655 ou d'un des reclones de l'anticorps, lequel procédé comprend l'inoculation d'un animal au moyen d'un matériel contenant l'antigène, l'obtention de cellules de l'animal productrices d'anticorps, la fusion des cellules productrices d'anticorps et de cellules néoplasiques pour produire des hybridomes, la sélection de l'hybridome, la production de clones des hybridomes choisis et la collecte de l'anticorps ainsi produit.

2.  Un procédé selon la revendication 1 où les cellules productrices d'anticorps sont des cellules de rate de souris BALB/C.

3.  Un procédé selon l'une quelconque des revendications précédentes où le matériel contenant l'antigène dérive de la lignée cellulaire MCF-7 de carcinome mammaire humain.

4.  Un procédé selon l'une quelconque des revendications précédentes où les cellules de tissus néoplasiques sont les cellules de myélome de la lignée PAI.

5.  Un procédé selon l'une quelconque des revendications précédentes où les clones sont développés chez des animaux hôtes et l'anticorps est obtenu à partir du sérum du sang de ces animaux hôtes ou du liquide ascite de ces animaux hôtes.

6.  Un procédé selon l'une des revendications 1 à 4 ou les clones sont développés in vitro et l'anticorps est obtenu à partir des surnageants de culture des clones.

**Patentansprüche**
**Patentansprüche für folgende Vertragsstaaten : BE, CH, FR, GB, IT, LI, LU, NL, SE**

1.  Zellinie, hergestellt durch die Fusion einer Antikörper erzeugende Tierzelle und einer neoplastischen Zelle, **dadurch gekennzeichnet,**
    daß sie das Hybridom HB-8655 oder ein Antikörper-produzierender Klonierungsabkömmling (Reclone) davon ist.

2.  Zellinie nach Anspruch 1, worin die Antikörper-erzeugende Zelle abgeleitet ist von einem Nager.

3.  Zellinie nach Anspruch 2, worin die Antikörper-erzeugenden Zellen Nager-Milzzellen oder Nager-Lymphknotenzellen sind.

4. Zellinie nach Anspruch 3, worin die Antikörper-erzeugenden Zellen Balb/c-Maus-Milzzellen sind.

5. Zellinie nach einem der vorhergehenden Ansprüche, worin die neoplastische Zelle eine Zelle ist, die keine Antikörper produziert.

6. Monoklonaler Antikörper,
   **dadurch gekennzeichnet,**
   daß er erhalten wird aus HB-8655 Hybridomkulturen oder Antikörper-produzierenden Klonierungsabkömmlingen davon.

7. Verfahren zur Herstellung von monoklonalen Antikörpern gemäß Anspruch 6, wobei das Verfahren Impfen eines Tieres mit von der menschlichen Mammakarzinom-Zellinie MCF-7 abgeleitetem Material umfaßt.

**Patentansprüche für folgenden Vertragsstaat : AT**

1. Verfahren zur Herstellung eines monoklonalen Antikörpers, welcher
   **gekennzeichnet ist,**
   daß er erhalten wird aus HB-8655 Hybridomkulturen oder Antikörper erzeugenden Klonierungsabkömmlingen davon, wobei das Verfahren das Impfen eines Tieres mit Material umfaßt, das das Antigen enthält, Gewinnen der Antikörper-erzeugenden Zellen aus dem Tier, Fusionieren der Antikörper-erzeugenden Zellen und neoplastischen Zellen, um Hybridome herzustellen, Selektionieren der Hybridome, Produzieren von Klonen der selektionierten Hybridome und Sammeln der Antikörper, die davon hergestellt werden.

2. Verfahren nach Anspruch 1, worin die Antikörper-erzeugenden Zellen BALB/c-Maus-Milzzellen sind.

3. Verfahren nach einem der vorhergehenden Ansprüche, worin das Antigen-enthaltende Material abgeleitet ist von der menschlichen Mammakarzinom-Zellinie MCF-7.

4. Verfahren nach einem der vorhergehenden Ansprüche, worin die neoplastischen Gewebezellen solche der Myelom-Zellinie PAI sind.

5. Verfahren nach einem der vorhergehenden Ansprüche, worin die Klone in Wirtstieren herangezogen werden und der Antikörper erhalten wird aus dem Blutserum des Wirtstieres oder der Ascitesflüssigkeit des Wirtstieres.

6. Verfahren nach einem der Ansprüche 1 bis 4, worin die Klone in vitro herangezogen werden und der Antikörper aus den Kulturüberständen der Klone erhalten wird.